# EUROPEAN PATENT SPECIFICATION

(11) **EP 0 441 978 B1**
(45) Date of publication and mention of the grant of the patent: **15.06.1994**
(21) Application number: 90912952.0
(22) Date of filing: 30.08.1990
(51) Int. Cl.: A61B 17/36

(54) **DEVICE FOR RADIATING LASER BEAMS**
VORRICHTUNG ZUM AUSSENDEN VON LASERSTRAHLEN
DISPOSITIF D'IRRADIATION DE FAISCEAUX LASER

(30) Priority: 01.09.1989 JP 227092/89
(43) Date of publication of application: 21.08.1991
(73) Proprietor: S.L.T. JAPAN CO, LTD., Shinjuku-ku, Tokyo 162 (JP)
(72) Inventor: DAIKUZONO, Norio 381, Kofudai 4-chome, Chiba-ken 290-02 (JP)
(74) Representative: Bloch, Gérard
(86) International application number: PCT/JP90/01108
(87) International publication number: WO 91/03206

(56) References cited:
- EP-A- 0 070 459
- DE-A- 2 821 376
- JP-U- 202 815
- JP-Y-10 648

## Description

### TECHNICAL FIELD

This invention relates to a laser light emitter in use for excision of a prominence of living tissue of animal organisms.

### PRIOR ART

It is known that a high frequency scalpel is used in excision of a prominence of living tissue as a prostate. For example, a high frequency scalpel has a hexagonal wire 51 (in general, it is called snare) projecting in front of a holder 50 as shown in Fig 11, and high frequency current is flown into the wire 51.

When such apparatus for excision is used, said wire 51 is so positioned that a prominence is disposed within the wire 51 so as to cauterize to remove the prominence by heat.

However, this kind of a high frequency snare has a disadvantage fundamentally. Namely, when a prominence, that is an affected part, is removed under the presence of physiological salt solution, electricity flowing through physiological salt solution occasionally gives a shock to the human body or a burn near the affected part.

So an operator cannot but substitute physiological salt solution by distilled water in medical treatment. And it is impossible to remove a prominence with stopping of bleeding.

DE-A-28 21 376 teaches a laser light emitter for excision of a prominence of an animal organism comprising a holding portion and an excision means forming a transmission path for the laser light, wherein a part of said excision means comprises a laser light emitting portion formed as an uncovered,portion of said excision means, emitting laser light at least in the direction (C) of the holding portion, the remaining part of said excision means comprises a light emission-intercepting portion situated in the side opposite to said direction of the holding portion in order to shut off the laser light emission, and said laser light emitting portion is optically connected with a laser light generator.

However, this prior art laser light emitter cannot exert a good excision under the presence of physiological salt solution and with a good control of bleeding.

### DISCLOSURE OF THE INVENTION

In order to solve above mentioned problems, the present invention relates to a laser light emitter of the above mentioned type, characterized by the fact that the uncovered portion is an elongated side portion along the length of the excision means.

Moreover, in the prefered embodiment of the invention, said laser light emitter is made of an optical fiber covered with a metal member, the core of said optical fiber is exposed at said uncovered portion so as to form the laser emitting portion, and the remaining part of said excision means is covered with said metal member to form the laser light emission-intercepting portion.

The other preferred embodiments are according to the dependent claims 3 to 9.

Accordingly, safe excision of the prominence can be made without giving a shock to the human body or a burn, if laser light is emitted under the presence of physiological salt solution.

In addition, it is possible to remove the prominence with control of bleeding by regulating quantity of laser light emission and the ability of hemostasis by laser light. Therefore a laser light emitter according to the present invention is so effective for excision of hemorrhagic organism.

### BRIEF DESCRIPTION OF THE DRAWINGS

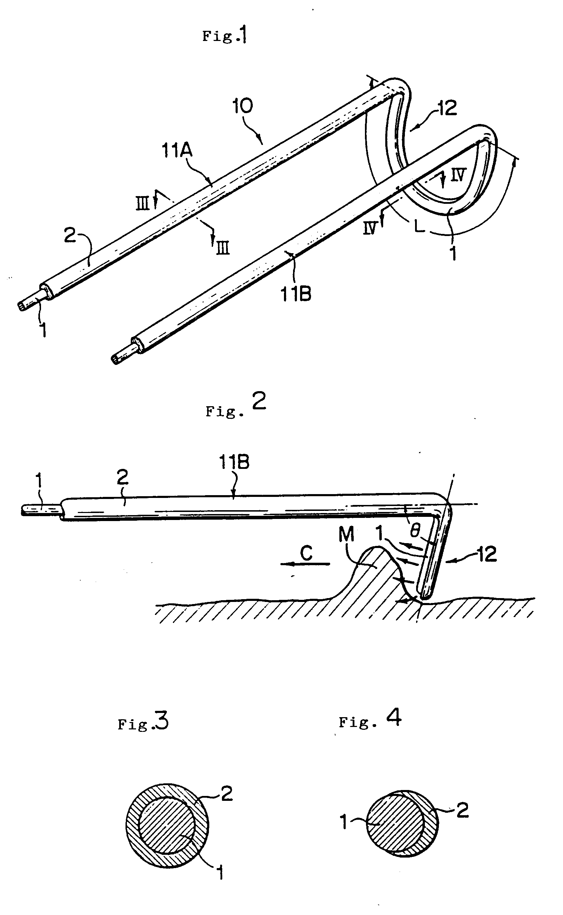

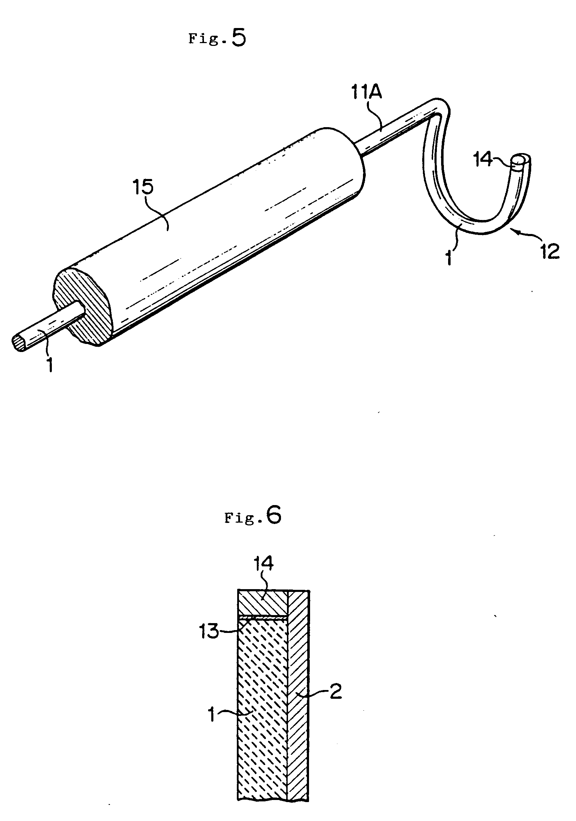

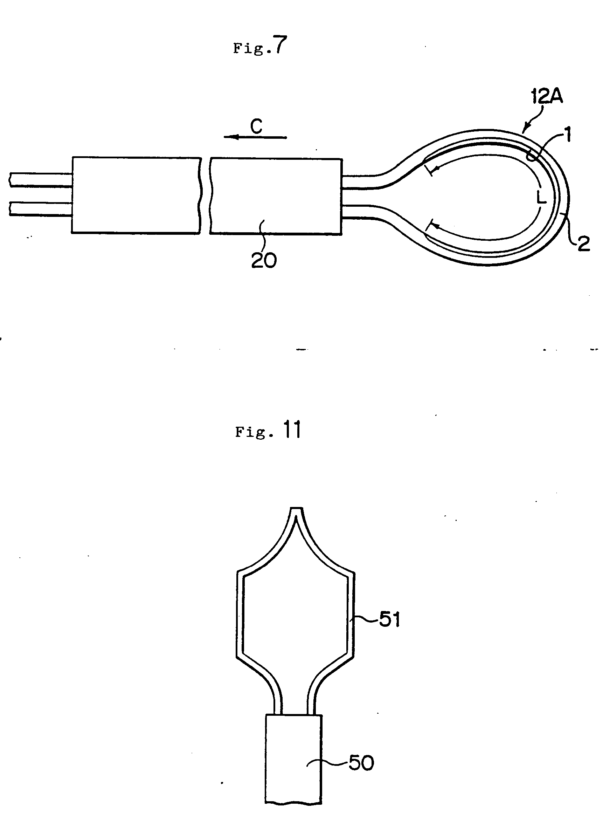

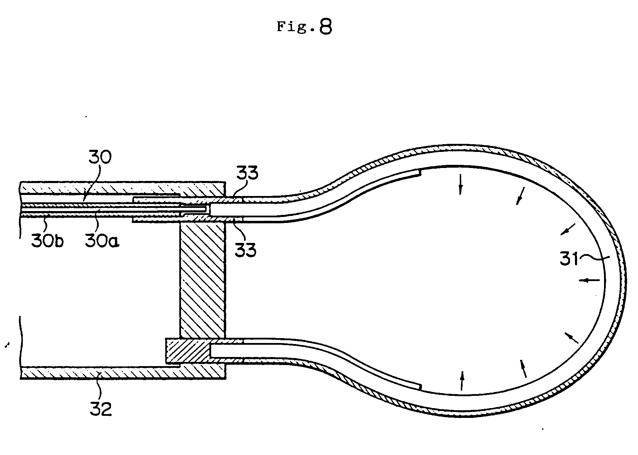

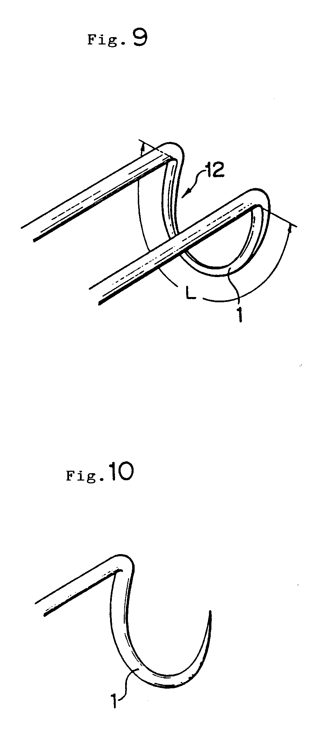

Fig.1 is a perspective view of the first embodiment of a laser light emitter according to the present invention; Fig.2 is a front view of the embodiment of Fig.1; Fig.3 is a sectional view of the embodiment of Fig.1 taken on line III - III; Fig.4 is a sectional view of the embodiment of Fig.1 taken on line IV - IV; Fig.5 is a perspective view of the second embodiment of a laser light emitter according to the present invention; Fig.6 is a vertical sectional view of a part of the second embodiment; Fig.7 is a plan view of the third embodiment of a laser light emitter according to the present invention; Fig.9 and Fig.10 are perspective views of still other embodiments; Fig.8 is a vertical sectional view of another embodiment, wherein laser light is emitted through a light-transmissible ceramic material provided at the end of optical fiber; Fig.11 is a plan view of a conventional apparatus.

### THE BEST MODE TO CARRY OUT THE INVENTION

The present invention is described more particularly hereinafter.

Fig.1-Fig4 show the first embodiment. An optical fiber 1 is covered with a metal member, for example a stainless-steel tube 2.

An instrument for excision 10 provides parallel holding portions 11A,11B and an open-loop portion 12 continuously connected with the holding portion 11A and the holding portion 11B.

The open-loop portion 12 is projecting downward in the shape of letter U from the ends of the holding portions 11A,11B. Either side of optical fiber for example the other end of the optical fiber 1 covered with the holding portion 11A is optically connected with a laser light generator (not illustrated).

On the other hand, a part of the open-loop portion 12 composes a laser light emitting portion L which is capable of emitting laser light in the direction C for excision the prominence M, and the rest part of the open-loop portion 12 composes a emission-intercepting portion in the opposite side to the direction C for excision in order to shut off the laser light emission.

To be concrete, the open-loop portion 12 is formed as an uncovered portion by a stainless-steel tube 2 on the emitting portion L and the core of the optical fiber 1 is exposed at the uncovered portion so as to form the emitting portion L, and the rest part of open-loop portion 12 is covered with the stainless-steel tube 2 to form the laser light emission-intercepting portion in the present invention.

In such apparatus, a laser light generated from the light generator is transmitted into the optical fiber 1. Wherein laser light is so covered with the stainless-steel tube 2 that laser light is not emitted at the covered portion. Accordingly, a part of the stainless-steel tube 2 on the emitting portion L is so opened in the direction C for excision that a laser light is emitted from the exposed portion of the core of the optical fiber 1 in Fig.2. Laser light is irradiated to the prominence M, and excision is done from the root of the prominence M by the excision ability of laser light. An operator generally holds the instrument for excision in the present invention on the holding portion 11A, 11B, and then moves succesively the instrument in the direction C for excision to exert excision.

In the above-described embodiment, the core of the optical fiber 1 is directly covered with the stainless-steel tube 2, however the core of the optical fiber 1 may be covered with clad. Wherein the core of the optical fiber 1 needs to be exposed by removing of the clad on at least the emitting portion L.

In the first embodiment, the angle ϑ defined by the plane containing a straight portion 11A,11B of the holding portion and a plane containing the open-loop portion is 45-120°, preferably 65-85 ° .

In the first embodiment, the parallel holding portion 11A,11B are formed to be hold, however only the holding portion 11A is formed and the emitting portion L is capable of being formed at the end of the holding portion 11A in Fig.5. The part designated by numeral 15 is a holder provided if needed for making easy to hold.

Moreover, according to the second embodiment in Fig.5 and Fig.6, a laser light reflecting portion 13 is provided at the end of the emitting portion L, wherein the reflecting portion 13 prevents laser light from being emitted at the end of the optical fiber 1 and laser light is capable of being emitted only at the side of the optical fiber 1. The reflecting portion 13 is providing a supporting member 14 continuously at the end of the stainless-steel tube 2 and the reflecting portion 13 is capable of being made of a reflecting material as a layer made by a gold plating positioned inside the supporting member 14.

Further, the emitting portion L is formed in U-shape in the above-described embodiments, when it is observed from the left side in Fig.2. However it may be a circle or a trapezium or a rectangle (the top of every shape needs to remain discontinuous).

On the other hand, Fig.7 shows a plan view of the third embodiment, and an open-loop porton 12A is formed at the end of a holder 20. A part of stainless-steel tube 2 is broken away and the core of the optical fiber 1 is exposed as shown in the first embodiment.

In the third embodiment, the open-loop portion 12 is so positioned that the prominence is disposed within the open-loop portion 12A like a conventional snare, and then the open-loop portion 12A is moved in the direction C to exert excision by laser light irradiation.

In the third embodiment, the holder 20 can be omitted.

On the other hand, in the above-described embodiments, the emitting portion is formed on almost the whole of the open-loop portion 12 or 12A, however in the first embodiment the emitting portion can be formed only at the bottom of the U-shaped portion and in the third embodiment the emitting portion can be formed only on the right half of the open-loop portion.

Moreover, in the above-described embodiments, the core of the optical fiber is covered with a metal member for example stainless-steel tube from the point of view of strength. However, if so much strength is not needed, a light emitter can be formed of single optical fiber covered with a clad, wherein the clad is partly broken away and the core is exposed to form the emitting portion. In this case, the clad is equivalent to the light emission-intercepting portion in the present invention.

Moreover, in a modification of the third embodiment illustrated in Fig.8, an emitting member 31 made of a light-transmissible ceramic for example bent into ring-shape having circular cross section is provided at the end of an optical fiber 30 having a core 30a and a clad 30b, and laser light exited at the end of the core 30a of the optical fiber 30 enters into the emitting member 31, and then laser light is capable of being emitted from the emitting member 31. The part designated by numeral 32 is a holder and the part designated by numeral 33 is the instrument for connecting. Such method of the indirect incidence is applicable in the first embodiment and the second embodiment.

On the other hand, as shown in Fig.9 and Fig.10 respectively corresponding to Fig.1 and Fig.5, if laser light is wanted to be emitted more concentratedly to the direction for excision, the core 1 is to be so formed that its diameter of a circular cross-section gradually gets thinner from the holding portion (11a) onwards. Then the quantity of emitted laser light increases at the diminished portion as with the case of a slender conic probe, so the laser light can be led to the direction for excision more effectively.

On the other hand, the surface of a laser light emitting portion can be provided with the surface layer for diffusing of laser light if needed. A laser light absorbing particle as carbon or a laser light diffusing particle as silica having larger refractive index than that of an emitting member as a core material can be utilized. And the surface of a laser light emitting portion can be provided with a roughened surface to raise diffusing.

Moreover, a laser light emitter in the present invention is capable of being used in an operation by using endoscope as well as a surgical operation.

### INDUSTRIAL UTILIZATION

As a result, according to the present invention, it is possible to remove the prominence under the presence of physiological salt solution with controlling bleeding without giving a shock to the human body.

## Claims

1. A laser light emitter for excision of a prominence of an animal orqanism comprising a holdinq portion (11a) and an excision means (12) forming a transmission path for the laser light, wherein a part of said excision means comprises a laser light emitting portion (L,1;31) formed as an uncovered portion of said excision means, emitting laser light at least in the direction (C) of the holding portion, the remaining part of said excision means comprises a light emission-intercepting portion (2) in order to shut off the laser light emission, and said laser light emitting portion is optically connected with a laser light generator, characterized by the fact that the uncovered portion (1) is an elongated side portion along the length of the excision means.

2. A laser light emitter according to claim 1, wherein said laser light emitter is made of an optical fiber (1) covered with a metal member (2), the core of said optical fiber (1) is exposed at said uncovered portion so as to form the laser emitting portion, and the remaining part of said excision means is covered with said metal member (2) to form the laser light emission-intercepting portion.

3. A laser light emitter according to claim 1, wherein said laser light emitter includes at least one straight portion (11a) connected to an open-loop portion (12).

4. A laser light emitter according to claim 3, wherein the open-loop portion (12) is situated in a plane defining an angle between 45° and 120° with a plane containing the straight portion (11a).

5. A laser light emitter according to claim 1, wherein the excision means (12) is U-shaped.

6. A laser light emitter according to claim 1, wherein the laser light emitting portion (1) has a circular cross-section of which the diameter gradually gets thinner from said holding portion (11a) onwards such that it concentrates light.

7. A laser light emitter according to claim 1, wherein the laser light emitting portion comprises a light-transmission ceramic (31).

8. A laser light emitter according to claim 1, wherein the laser light emitting portion comprises an optical fiber (1) covered with a clad.

9. A laser light emitter according to claim 1, wherein a laser light reflecting portion (13) is provided at the end of the laser light emitting portion (1) for preventing laser light from being emitted at the end of the laser light emitting portion (1).

## Patentansprüche

1. Vorrichtung zum Aussenden von Laserlicht zur Ausschneidung eines vorstehenden Teils eines tierischen Organismus, umfassend einen Halteteil (11a) und ein Mittel zum Ausschneiden (12), die einen Übertragungsweg für das Laserlicht bilden, bei der ein Teil der Ausschneidemittel einen Laserlicht emittierenden Bereich (L, 1; 31) umfaßt, der als ein freiliegender Bereich dieser Ausschneidemittel gebildet ist und Laserlicht mindestens in Richtung (C) des Halteteils aussendet, wobei der übrige Teil der Ausschneidemittel einen die Lichtemission unterbrechenden Bereich (2) umfaßt, um die Laserlichtemission auszuschalten, und der Laserlicht emittierende Bereich optisch mit einem Laserlicht-Generator verbunden ist, dadurch gekennzeichnet, daß der freiliegende Bereich (1) ein länglicher seitlicher Teil entlang der Länge der Ausschneidemittel ist.

2. Vorrichtung zum Aussenden von Laserlicht nach Anspruch 1, bei der diese Laserlicht-Aussendevorrichtung aus einer Lichtleitfaser (1) besteht, die von einem Metallelement (2) umhüllt ist, wobei der Kern dieser Lichtleitfaser (1) in dem freiliegenden Bereich offen liegt, um den Laserlicht emittierenden Bereich zu bilden, und der übrige Teil der Ausschneidemittel von dem Metallelement (2) umhüllt ist, um den die Lichtemission unterbrechenden Bereich zu bilden.

3. Vorrichtung zum Aussenden von Laserlicht nach Anspruch 1, bei der diese Laserlicht-Aussendevorrichtung mindestens einen geraden Teil (11a) umfaßt, der an einen eine offene Schleife bildenden Abschnitt (12) anschließt.

4. Vorrichtung zum Aussenden von Laserlicht nach Anspruch 3, bei der dieser eine offene Schleife bildende Abschnitt (12) in einer Ebene liegt, die einen Winkel zwischen 45° und 120° mit einer Ebene, welche den geraden Teil (11a) enthält, definiert.

5. Vorrichtung zum Aussenden von Laserlicht nach Anspruch 1, bei der die Ausschneidemittel (12) U-förmig sind.

6. Vorrichtung zum Aussenden von Laserlicht nach Anspruch 1, bei der der Laserlicht emittierende Bereich (1) einen kreisförmigen Querschnitt besitzt, dessen Durchmesser von dem Halteteil (11a) an allmählich dünner wird, so daß er das Licht konzentriert.

7. Vorrichtung zum Aussenden von Laserlicht nach Anspruch 1, bei der der Laserlicht emittierende Bereich eine lichtübertragende Keramik (31) umfaßt.

8. Vorrichtung zum Aussenden von Laserlicht nach Anspruch 1, bei der der Laserlicht emittierende Bereich eine ummantelte Lichtleitfaser (1) umfaßt.

9. Vorrichtung zum Aussenden von Laserlicht nach Anspruch 1, bei der ein Laserlicht reflektierender Teil (13) am Ende des Laserlicht emittierenden Bereichs (1) vorgesehen ist, um zu verhindern, daß Laserlicht am Ende des Laserlicht emittierenden Bereichs (1) ausgesendet wird.

## Revendications

1. Emetteur de lumière laser pour l'excision d'une proéminence d'un organisme animal, comprenant une partie de maintien (11a) et des moyens d'excision (12) formant un trajet de transmission pour la lumière laser, dans lequel une partie desdits moyens d'excision comprend une partie d'émission de lumière laser (L, 1 ; 31) réalisée sous la forme d'une partie découverte desdits moyens d'excision, émettant une lumière laser au moins dans la direction (C) de la partie de maintien, la partie restante desdits moyens d'excision comprend une partie (2) d'interception d'émission de lumière servant à interrompre l'émission de lumière laser et ladite partie d'émission de lumière laser est reliée optiquement à un générateur de lumière laser, caractérisé par le fait que la partie découverte (1) est une partie latérale allongée s'étendant sur la longueur des moyens d'excision.

2. Emetteur de lumière laser selon la revendication 1, dans lequel ledit émetteur de lumière laser est constitué d'une fibre optique (1) recouverte d'un corps métallique (2), l'âme de ladite fibre optique (1) étant exposée au niveau de la partie découverte de manière à former la partie d'émission de lumière et la partie restante desdits moyens d'excision est recouverte par ledit corps métallique (2), pour former la partie (2) d'interception d'émission de lumière laser.

3. Emetteur de lumière laser selon la revendication 1, dans lequel ledit émetteur de lumière laser comprend au moins une partie rectiligne (11a) reliée à une partie en boucle ouverte (12).

4. Emetteur de lumière laser selon la revendication 3, dans lequel la partie en boucle ouverte (12) est située dans un plan formant un angle compris entre 45° et 120° avec un plan contenant la partie rectiligne (11a).

5. Emetteur de lumière laser selon la revendication 1, dans lequel les moyens d'excision (12) présentent une forme en U.

6. Emetteur de lumière laser selon la revendication 1, dans lequel la partie d'émission de lumière laser (1) présente une section transversale circulaire dont le diamètre diminue progressivement depuis ladite partie de maintien (11a), de manière à concentrer la lumière.

7. Emetteur de lumière laser selon la revendication 1, dans lequel la partie d'émission de lumière laser comprend un matériau céramique de transmission de lumière (31).

8. Emetteur de lumière laser selon la revendication 1, dans lequel la partie d'émission de lumière laser comprend une fibre optique (1) recouverte d'une gaine.

9. Emetteur de lumière laser selon la revendication 1, dans lequel une partie de réflexion de lumière laser (13) est prévue à l'extrémité de la partie d'émission de lumière laser (1), afin d'empêcher l'émission de lumière laser à l'extrémité de la partie d'émission de lumière laser (1).
